# EUROPEAN PATENT SPECIFICATION

(11) **EP 3 960 151 B1**
(45) Date of publication and mention of the grant of the patent: **23.04.2025**
(21) Application number: 20382765.4
(22) Date of filing: 27.08.2020
(51) Int. Cl.: A61K 8/81, B05C 17/005, C08F 22/32, C08F 222/32, C08L 33/12, C09J 4/06, A61L 24/04, A61L 24/06

(54) **PHOTOCURABLE CYANOACRYLATE COMPOSITION WITH CONTROLLED EXOTHERM**
PHOTOHÄRTBARE CYANOACRYLATZUSAMMENSETZUNG MIT KONTROLLIERTER EXOTHERMIE
COMPOSITION DE CYANOACRYLATE PHOTODURCISSABLE À EXOTHERME CONTRÔLÉ

(43) Date of publication of application: 02.03.2022
(73) Proprietor: Cuantum Medical Cosmetics, S.L., 08193 Cerdanyola del Vallès (Barcelona) (ES)
(72) Inventor: AGUILERA CORROCHANO, Jordi, 08202 Sabadell (Barcelona) (ES); ORTEGO HUGUET, Sara, 07712 Maó (Illes Balears) (ES); SÁEZ FRAILE, Rubén, 08021 Barcelona (ES); CLOSA FERRER, Guillem, 08172 Sant Cugat del Vallès (Barcelona) (ES); TORRALBA MALDONADO, Daniel, 08225 Terrassa (Barcelona) (ES); PIZÀ JUAN-MUNS, Feliu, 082020 Sabadell (Barcelona) (ES); PUJOL NOGUERA, Ferran, 08635 Sant Esteve Sesrovires (Barcelona) (ES)
(74) Representative: González Poveda, Sara

(56) References cited:
- WO-A1-03/065841
- WO-A1-2020/085334
- JP-A- 2000 273 402
- JP-B2- 4 998 671
- US-A- 5 824 180

## Description

### Field of the invention

The present invention relates to the field of photocurable cyanoacrylate compositions, in particular cyanoacrylate compositions with controlled exotherm, and the use of said compositions for bonding substrates, in particular as bioadhesive and biosealant.

### Background art

The surgical suture is used to hold body tissues together after injury or surgery. Sutures are typically applied using a needle with an attached piece of thread and are secured with surgical knots. The task of the suture in surgery is to seal the separated area and to facilitate the natural healing process. However, suturing involves additional trauma to the wound and sometimes it is unfeasible, for instance when a lot of biological tissue has been lost or when the tissue is weakened. Therefore, the search for alternatives to conventional sutures is a field of interest in surgery.

Adhesive bonding is an alternative in front of suturing. As disclosed in International patent application WO-A-02/09784, adhesive bonding shows some advantages: tissue bonding is achieved quickly; forces tending to separate the edges of the wound or incision are distributed more evenly; the additional trauma which suturing involves is avoided; the risk of microbe contamination is reduced by sealing the wound or incision entirely; using adhesives allows filling of intermediate areas of the wound that are difficult to access; and surplus adhesive applied comes away from the healed wound.

Cyanoacrylates (CAs) are esters of cyanoacrylic acid, which are used as strong fast-acting adhesives with industrial, medical, and household uses. These compounds rapidly polymerize and cure by the action of water present on adherend substrates to extremely tenaciously bond the substrates to each other in a short time period. However, as disclosed in European patent application EP-A-0769721, cyanoacrylate adhesives have a drawback that the curing is very slow when the gap between adherends is wide, when the adhesive applied has overflown from the bonding part, or when the adhesive applied is not sandwiched between a pair of adherends as in coating. To solve said problem, photocurable cyanoacrylate compositions have been proposed in the state of the art, for example, in above-mentioned EP-A-0769721, International patent applications WO-A-98/38260 and WO-A-03/065841, and in Japanese patent application JP-A-2003-277422.

However, one of the drawbacks of cyanoacrylate adhesives is that polymerisation is an exothermic reaction that can lead to localised high temperatures causing discomfort and even damage, as disclosed in Jarrett et al., Tissue adhesives and sealants for surgical applications, in Joining and assembly of medical materials and devices, Y. (Norman) Zhou and Mark D. Breyen (Eds.), Woodhead Publishing Ltd., Cambridge, 2013, pages 449-490.

In the state of the art, several technical solutions have been proposed to control the exothermic polymerisation reaction.

US patents US5219645 and US5319011 disclose the use of an organotin compound to polymerize a cyanoacrylate composition without any significant exotherm.

US patent US5369144 discloses an adhesive composition comprising 2-methoxyisopropyl 2-cyanoacrylate, an alkyl (meth)acrylate and a fluorinated alkyl (meth)acrylate to get a sufficiently lowered exothermic temperature.

US patent US6010714 discloses a composition useful in the formulation of biomedical adhesives and sealants, which comprises a monomer component, such as a cyanoacrylate ester, and a heat dissipating agent to reduce the temperature increase due to the polymerisation of the monomer.

In above-mentioned WO-A-02/09784 it is disclosed an adhesive composition comprising a specific cyanoacrylate and a specific acrylate, which is histocompatible and suitable for joining biological tissue, even in ophthalmic surgery. Although it refers to the exothermic polymerisation reaction of cyanoacrylates, it is not disclosed any effect of the composition on the exothermic process.

International patent application WO-A-03/008002 refers to the exothermic polymerisation reaction of cyanoacrylates, wherein the generated heat can result in patient discomfort, and discloses a wound sealing device, which is used to apply a sealing adhesive (e.g. cyanoacrylate composition).

International patent application WO-A-2006/023605 discloses a tissue adhesive comprising specific amounts of a cyanoacrylate polymer; a plasticizer and a mixture of first and second cyanoacrylate monomers, resulting a lower temperature in the adhesive and surrounding tissues during polymerisation.

International patent application WO-A-2010/008822 discloses a sterilized cyanoacrylate adhesive composition including a cyanoacrylate composition and a cure speed enhancer (e.g. a crown ether), wherein said sterilized cyanoacrylate adhesive composition shows an enhanced cure speed for medical use and that, once cured, exhibits improved permeability and mechanical properties such as, for example, wound closure strength, overlap shear strength, peel adhesive strength, and flexibility. Although it refers to the exothermic polymerisation reaction of cyanoacrylates, it is not disclosed any effect of the composition on the exothermic process.

International patent application WO-A-2010/014641 discloses an adhesive composition comprising one or more polymerizable cyanoacrylate monomers, a polymerisation initiator, which includes a quaternary ammonium salt, and a polymerisation accelerator, which includes a trihydroxy tertiary amine. A controlled exotherm is achieved by combined said initiator and said accelerator.

International patent application WO-A-2010/114779 discloses an adhesive composition comprising one or more polymerizable cyanoacrylate monomers and a specific multifunctional hydroxyamine compound, additive which reduces the amount of heat released during the polymerisation of the cyanoacrylate monomer. Other optional components may be present in the composition including, for example, heat dissipating agents.

International patent application WO-A-2012/136639 discloses a composition showing reduced exothermic polymerisation, which comprises a combination of specific alkyl cyanoacrylates, alkyl carboxyacrylates and alkyl acrylates.

German patent application DE-A-102012216425 discloses an adhesive composition comprising cyanoacrylate monomers and a polymer selected from the group comprising polyvinylpyrrolidone, polyvinylpyrrolidone derivative(s), and copolymers comprising 1-vinyl-2-pyrrolidone as monomer. By spraying said components, the exothermic reaction occurring by polymerizing can be reduced.

Thus, there is still a need to provide an adhesive that combines the speed and multi-substrate bonding attributes of CAs with controlled exotherm.

### Subject-matter of the invention

The subject-matter of the present invention is a photocurable cyanoacrylate composition.

Another aspect of the invention relates to a syringe or a cartridge comprising said composition.

Another aspect of the invention relates to the use of said composition for bonding substrates.

Another aspect of the invention relates to the use of said composition as nail coating.

Another aspect of the invention is a method for bonding substrates using said composition.

Another aspect of the invention is a photocurable cyanoacrylate composition for use as bioadhesive and biosealant.

### Detailed description of the invention

The object of the present invention is a photocurable cyanoacrylate composition comprising:
a) at least 40 wt.% of a cyanoacrylate monomer,
b) between 0.0055 wt.% and 0.0295 wt.% of ferrocene,
c) between 0.1 wt.% and 0.8 wt.% of a benzoyl phosphine oxide as photoinitiator, preferably selected from phenyl bis(2,4,6-trimethylbenzoyl)phosphine oxide (BAPO), diphenyl (2,4,6-trimethylbenzoyl)phosphine oxide (TPO), benzoyl-diphenylphosphine oxide (BDPO), 2,4,6-trimethylbenzoyl-methoxy-phenylphosphine oxide (TMMPO), phthaloyl-bis(diphenylphosphine oxide) (PBDPO), 2,6-difluoro benzoyl-diphenylphosphine oxide (DFDPO), and mixtures thereof, more preferably selected from phenyl bis(2,4,6-trimethylbenzoyl)phosphine oxide (BAPO), diphenyl (2,4,6-trimethylbenzoyl)phosphine oxide (TPO), and mixtures thereof, and
d) between 0.05 wt.% and 1.5 wt.% of a radical polymerisation inhibitor,
wherein the wt.% is based on the total weight of the composition.

The inventors of the present invention have developed a photocurable cyanoacrylate composition, which shows a controlled exotherm with reduced temperature increase compared to the prior art, which surprisingly performs with better T-peel adhesive strength in PVC substrates than analogous formulations of prior art without controlled exotherm. The composition of the invention further provides unexpected advantages as tissue adhesive, which have demonstrated greater duration on the skin as wound sealant than other commercially available tissue adhesives showing no on-demand photoinitiated polymerisation.

The photocurable composition of this application retain the benefits and advantages of traditional cyanoacrylate compositions, while curing trough at least a photo-induced polymerisation mechanism, providing benefits and advantages to the bonding.

Throughout the present description and in the claims, the expressions in singular preceded by the articles "a" or "the" are understood to also include, in a broad manner, the reference to the plural, unless the context clearly indicates the contrary.

In the context of the present invention, it is understood that the term "about" referred to a determined value indicates that a certain variation for said value is accepted, generally of +/- 5 %.

The ranges disclosed in this description include both the lower and the upper limit thereof.

### Cyanoacrylate monomer

The composition comprises a cyanoacrylate monomer, preferably selected from the group consisting of 2-methoxyethyl 2-cyanoacrylate, 2-ethoxyethyl 2-cyanoacrylate, ethyl 2-cyanoacrylate, *n*-propyl 2-cyanoacrylate, iso-propyl 2-cyanoacrylate, *n*-butyl 2-cyanoacrylate, *sec*-butyl 2-cyanoacrylate, iso-butyl 2-cyanoacrylate, *tert*-butyl 2-cyanoacrylate, *n*-pentyl 2-cyanoacrylate, neopentyl 2-cyanoacrylate, 1-ethylpropyl 2-cyanoacrylate, 1-methylbutyl 2-cyanoacrylate, *n*-hexyl 2-cyanoacrylate, *n*-heptyl 2-cyanoacrylate, *n*-octyl 2-cyanoacrylate, *2*-octyl 2-cyanoacrylate, 2-ethylhexyl 2-cyanoacrylate, and mixtures thereof.

Preferably, the cyanoacrylate monomer is selected from the group consisting of ethyl 2-cyanoacrylate, 2-methoxyethyl 2-cyanoacrylate, *n*-butyl 2-cyanoacrylate, *n*-hexyl 2-cyanoacrylate, 2-octyl 2-cyanoacrylate and mixtures thereof. More preferably, the cyanoacrylate monomer is selected from the group consisting of *n-*butyl 2-cyanoacrylate, *n*-hexyl 2-cyanoacrylate, *2*-octyl 2-cyanoacrylate and mixtures thereof. In a preferred embodiment, the cyanoacrylate monomer is *n*-butyl 2-cyanoacrylate. In another preferred embodiment, the cyanoacrylate monomer is *n*-hexyl 2-cyanoacrylate. In another preferred embodiment, the cyanoacrylate monomer is *2*-octyl 2-cyanoacrylate.

Such monomers can be prepared by methods known by the skilled in the art such as the method described, for example, in US 2,467,927. Some of them, such as, for example, ethyl 2-cyanoacrylate (ECA), *n*-butyl 2-cyanoacrylate (BCA), *n*-hexyl 2-cyanoacrylate (HCA), *2*-octyl 2-cyanoacrylate (OCA) and 2-methoxyethyl 2-cyanoacrylate (MECA) are commercially available.

In the composition of the invention, the content of cyanoacrylate monomer is usually higher than about 40 wt.%, preferably higher or equal to about 50 wt.%, preferably higher than about 60 wt.%, preferably higher than about 70 wt.%, preferably higher than about 75 wt.%, based on the total weight of the composition.

In an embodiment, the content of cyanoacrylate monomer is comprised between 65 wt.% and 95 wt.%, more preferably between 70 wt.% and 90 wt.%, more preferably between 75 wt.% and 87 wt.%, and yet more preferably between 78 wt.% and 85 wt.%, wherein the wt.% is based on the total weight of the composition.

In a preferred embodiment, the content of cyanoacrylate monomer is comprised between 65 wt.% and 90 wt.%, more preferably between 70 wt.% and 85 wt.%, more preferably between 75 wt.% and 80 wt.%, wherein the wt.% is based on the total weight of the composition.

In another preferred embodiment, the content of cyanoacrylate monomer is comprised between 75 wt.% and 95 wt.%, more preferably between 80 wt.% and 90 wt.%, and more preferably between 83 wt.% and 87 wt.%, based on the total weight of the composition.

### Ferrocene

Ferrocene is the organometallic compound named bis(*η*⁵-cyclopentadienyl) iron and defined by the formula Fe(C₆H₅) and CAS Registry Number 102-54-5. The molecule consists of two cyclopentadienyl rings bound on opposite sides of a central iron atom. It is commercially available through companies such as, for example, American elements or Sigma-Aldrich.

As disclosed in International patent application WO-A-2017/021785, ferrocene significantly improves the photosensitivity for polymerisation of ionically (anionically or zwitterionically) susceptible cyanoacrylate monomers, by interaction with the photolysis products from free radical photoinitiators.

The content of ferrocene in the composition is comprised between 0.0055 wt.% and 0.0295 wt.%, preferably between 0.0075 wt.% and 0.0275 wt.%, more preferably between 0.015 wt.% and 0.025 wt.%, and yet more preferably it is 0.02 wt.%, wherein the wt.% is based on the total weight of the composition. This range corresponds to between 55 ppm and 295 ppm of ferrocene, preferably between 75 ppm and 275 ppm, more preferably between 150 ppm and 250 ppm, and yet more preferably it is 200 ppm.

### Photoinitiators

A photoinitiator is a molecule that creates reactive species (free radicals, cations or anions) when exposed to radiation (UV or visible). Photoinitiators are well known in the state of the art and are usually selected from AIBN, benzoyl peroxide, and derivatives of phosphine oxides. Photochemistry of this type of compounds is disclosed, for example, in Slugget et al., (2,4,6-Trimethylbenzoyl)phosphine Oxide Photochemistry. A Direct Tome-Resolved Spectroscopic of Both Radical Fragments, J. Am. Chem Soc., 1995, 117, 5148-5153, in Meereis et al., BAPO as an alternative photoinitiator for the radical polymerisation of dental resins, Dental Materials, 2014, 30, 945-953, and in Ikemura et al., UV-VIS spectra and photoinitiation behaviours of acylphosphine oxide and bisacylphosphine oxide derivatives in unfilled, light-cured dental resins. Dental. Mat. J., 2008, 27(6), 765-774.

The composition comprises a benzoyl phosphine oxide as photoinitiator. Benzoyl phosphine oxides are preferably selected from phenyl bis(2,4,6-trimethylbenzoyl)phosphine oxide (BAPO), diphenyl (2,4,6-trimethylbenzoyl)phosphine oxide (TPO), benzoyl-diphenylphosphine oxide (BDPO), 2,4,6-trimethylbenzoyl-methoxy-phenylphosphine oxide (TMMPO), phthaloyl-bis(diphenylphosphine oxide) (PBDPO), 2,6-difluoro benzoyl-diphenylphosphine oxide (DFDPO), and mixtures thereof, more preferably selected from phenyl bis(2,4,6-trimethylbenzoyl)phosphine oxide (BAPO), diphenyl (2,4,6-trimethylbenzoyl)phosphine oxide (TPO), and mixtures thereof.

The content of photoinitiator in the composition is comprised between 0.1 wt.% and 0.8 wt.%, preferably 0.2 wt.% and 0.6 wt.%, more preferably between 0.24 wt.% and 0.44 wt.%, and yet more preferably between 0.3 wt.% and 0.4 wt.% of wherein the wt.% is based on the total weight of the composition. This range corresponds to between 1000 ppm and 8000 ppm, preferably between 2000 ppm and 6000 ppm, more preferably between 2400 ppm and 4400 ppm, and yet more preferably between 3000 ppm and 4000 ppm.

Benzoyl phosphine oxides are commercially available, or can be prepared as disclosed, for example, in Ikemura *et al., op.cit.* The photoinitiators BAPO and TPO are commercially available through companies, such as, for example, BASF or Rahn.

### Radical polymerisation inhibitor

Radical polymerisation inhibitors help to prevent premature polymerisation due any radical mechanism such as autoxidation or thermal polymerisation

Radical polymerisation inhibitors are preferably selected from the group consisting of 4-methoxyphenol, hydroquinone, hydroquinone monomethyl ether, hydroxytoluene butyl ether, hydroxyanisole butyl ether, 2,2'-methylenebis(4-methyl-6-*tert-*butylphenol), 4,4'-methylenbis (2,6-di-*tert*-butylphenol) and mixtures thereof; more preferably selected from the group consisting of hydroxyanisole butyl ether, 2,2'-methylenebis(4-methyl-6-*tert*-butylphenol), hydroxytoluene butyl ether, and mixtures thereof.

The composition comprises between 0.05 wt.% and 1.5 wt.%, preferably between 0.08 wt.% and 0.6 wt.%, more preferably between 0.09 wt.% and 0.5 wt.%, and yet more preferably between 0.1 wt.% and 0.4 wt.% of a radical polymerisation inhibitor, wherein the wt.% is based on the total weight of the composition.

Radical polymerisation inhibitors are commercially available through companies, such as, for example, TCI or Sigma-Aldrich.

### Acid stabilizing agents

In a preferred embodiment, the composition further comprises an acid stabilizing agent.

Acid stabilizing agents are inhibitors of the anionic polymerisation, and may be selected from the group consisting of Bronsted acids, Lewis acids, and mixtures thereof.

The acid stabilizing agent may be selected from the group consisting of boron trifluoride, boron trifluoride etherate, boron trifluoride dihydrate, trimethylsilyl triflate, sulphur dioxide, methanesulfonic acid, and mixtures thereof. In a more preferred embodiment, the acid stabilizing agent is selected from sulphur dioxide, boron trifluoride etherate, methanesulfonic acid, and mixtures thereof. In an embodiment, the composition further comprises a combination of sulphur dioxide and boron trifluoride etherate. In another embodiment, the composition further comprises a combination of sulphur dioxide, boron trifluoride etherate and methanesulfonic acid. In a more preferred embodiment, the composition further comprises between 0.0005 wt.% and 0.02 wt.%, preferably between 0.0006 wt.% and 0.015 wt.%, more preferably between 0.00065 wt.% and 0.01 wt.%, of sulphur dioxide, between 0 and 0.004 wt.%, preferably between 0.0001 wt.% and 0.003 wt.%, more preferably between 0.0005 wt.% and 0.0015 wt.% of boron trifluoride etherate, and between 0 and 0.005 wt.%, preferably between 0.0005 wt.% and 0.003 wt.%, more preferably between 0.00075 wt.% and 0.0015 wt.% of methanesulfonic acid, wherein the wt.% is based on the total weight of the composition.

In a preferred embodiment, the composition further comprises between 0.0005 wt.% and 0.02 wt.%, preferably between 0.0006 wt.% and 0.015 wt.%, more preferably between 0.00065 wt.% and 0.01 wt.%, of sulphur dioxide, and between 0.0001 wt.% and 0.003 wt.%, more preferably between 0.0005 wt.% and 0.0015 wt.% of boron trifluoride etherate, wherein the wt.% is based on the total weight of the composition.

In a preferred embodiment, the composition further comprises between 0.0005 wt.% and 0.02 wt.%, preferably between 0.0006 wt.% and 0.015 wt.%, more preferably between 0.00065 wt.% and 0.01 wt.%, of sulphur dioxide, and between 0.0001 wt.% and 0.003 wt.%, more preferably between 0.0005 wt.% and 0.0015 wt.% of boron trifluoride etherate, and between 0.0005 wt.% and 0.003 wt.%, more preferably between 0.00075 wt.% and 0.0015 wt.% of methanesulfonic acid, wherein the wt.% is based on the total weight of the composition.

### Additives

The composition may further contain one or more additives. Additives are selected, for example, from thickening agents, plasticizers, thixotropic agents, crosslinking agents, accelerating agents, and mixtures thereof.

In a preferred embodiment, the composition further comprises a combination of plasticizer and thickening agent.

### Thickening agent

A thickening agent is added to increase the viscosity of a cyanoacrylate-type adhesive composition. A suitable thickener or thickening agent for the composition may be selected from those which are compatible with the cyanoacrylate monomers contained therein.

Polymers can be used as thickeners, such as, for example, poly(meth)acrylates, acylated cellulose polymers, polyvinyl acetates, partially hydrolysed polyvinyl acetates, polyvinylpyrrolidones, vinyl acetate copolymers, copolymers of (meth)acrylates with butadiene and styrene, copolymers of vinyl chloride and acrylonitrile, copolymers of ethylene and vinyl acetate, and mixtures thereof.

Certain fumed silica fillers, optionally treated with polydialkylsiloxanes or trialkoxyalkylsilanes, can also be used as thickening agent.

In the scope of the invention, the thickening agent is selected from poly(meth)acrylates, acylated cellulose polymers, polyvinyl acetates, partially hydrolysed polyvinyl acetates, polyvinylpyrrolidones, vinyl acetate copolymers, copolymers of (meth)acrylates with butadiene and styrene, copolymers of vinyl chloride and acrylonitrile, copolymers of ethylene and vinyl acetate, fumed silica, fumed silica treated with polydialkylsiloxanes or trialkoxyalkylsilanes, and mixtures thereof.

Preferably, in the composition of the invention the thickener is selected from the group consisting of vinyl acetate copolymers and fumed silica.

Generally, the content of thickener is comprised between about 3 wt.% and about 20 wt.%, preferably between about 5 wt.% and about 18 wt.%, and more preferably between about 5 wt. % and about 15 wt.%, wherein the wt.% is based on the total weight of the composition.

These thickening agents are well known to the skilled person and have been described in the prior art.

Polymers suitable as thickening agent are commercially available through companies such as, for example, Wacker, Evonik or Lanxess.

Fumed silica is commercially available through companies such as, for example, Evonik.

### Plasticizer

The composition may comprise a plasticizer. Usually, the plasticizer is selected from organic esters such as, for example, acetates, carbonates, citrates, succinates, azelates, adipates, sebacates, stearates, myristates and phthalates.

Preferably the plasticizer is selected from triacetin, propylene carbonate, and mixtures thereof.

Typically, the content of the plasticizer in the composition is comprised between about 1 wt.% and about 20 wt. %, preferably between about 3 wt.% and about 15 wt. %, more preferably between about 5 wt.% and about 12 wt.%, and yet more preferably between about 8 wt.% and about 11 wt.%, wherein the wt.% is based on the total weight of the composition.

### Thixotropic agent

A thixotropic agent may be added to the composition to manage its rheological behaviour.

Preferably, the thixotropic agent is silica, which is available as fumed silica, optionally hydrophobized, and precipitated silica. In a more preferred embodiment, it is hydrophobized fumed silica. Silica may act both as thickening agent and as thixotropic agent.

Typically, the content of thixotropic agent in the composition is comprised between about 2 wt.% and about 10 wt.%, preferably between about 3 wt.% and about 8 wt.%, and more preferably between about 4 wt.% and about 7 wt.%, wherein the wt.% is based on the total weight of the composition.

Fumed silica is commercially available through companies such as, for example, Evonik.

### Crosslinking agents

The composition may further comprise a crosslinking agent, for example, tri- or difunctional monomeric crosslinking agents, to improve the cohesive strength of adhesive bonds formed therefrom.

Such crosslinking agents are known to the skilled person in the art, and are disclosed, for example in US patent 3,940,362, and commercially available through companies, such as, for example, IGM.

Examples of suitable crosslinking agents include alkyl bis(2-cyanoacrylates), triallyl isocyanurates, alkylene diacrylates, alkylene dimethylacrylates, bisphenol A alkoxylated diacrylate, bisphenol A epoxy diacrylate, trimethylolpropane trimethacrylate and trimethylolpropane triacrylate.

The content of the crosslinking agent is usually comprised between about 0.1 wt.% to about 50 wt.%, preferably between about 0.5 wt.% and about 40 wt.%, wherein the wt.% is based on the total weight of the composition.

### Accelerating agent

The composition may further comprise an accelerating agent.

A suitable accelerating agent or accelerator for the composition is a crown ether.

Typically, the content of the accelerating agent in the composition is comprised between about 0.01 wt.% and about 0.8 wt.%, preferably between about 0.05 wt.% and about 0.5 wt.%, and more preferably between about 0.1 wt.% and about 0.3 wt.%, wherein the wt.% is based on the total weight of the composition.

### Photocurable cyanoacrylate composition

The photocurable composition may be dispensed from packages, such as a syringe or from reservoir pots.

In a preferred embodiment, the photocurable cyanoacrylate composition comprises:
a) at least 40 wt.%, preferably between 65 wt.% and 95 wt.% of a cyanoacrylate monomer,
b) between 0.0055 wt.% and 0.0295 wt.% of ferrocene, preferably between 0.0075 wt.% and 0.0275 wt.%, more preferably between 0.015 wt.% and 0.025 wt.%, and yet more preferably it is 0.02 wt.%
c) between 0.1 wt.% and 0.8 wt.%, preferably between 0.2 wt.% and 0.6 wt.%, more preferably between 0.24 wt.% and 0.44 wt.%, and yet more preferably between 0.3 wt.% and 0.4 wt.% of a benzoyl phosphine oxide as photoinitiator, preferably selected from phenyl bis(2,4,6-trimethylbenzoyl)phosphine oxide (BAPO), diphenyl (2,4,6-trimethylbenzoyl)phosphine oxide (TPO), benzoyl-diphenylphosphine oxide (BDPO), 2,4,6-trimethylbenzoyl-methoxy-phenylphosphine oxide (TMMPO), phthaloyl-bis(diphenylphosphine oxide) (PBDPO), 2,6-difluoro benzoyl-diphenylphosphine oxide (DFDPO), and mixtures thereof, more preferably selected from phenyl bis(2,4,6-trimethylbenzoyl)phosphine oxide (BAPO), diphenyl (2,4,6-trimethylbenzoyl)phosphine oxide (TPO), and mixtures thereof,
d) between 0.05 wt.% and 1.5 wt.%, preferably between 0.08 wt.% and 0.6 wt.%, more preferably between 0.09 wt.% and 0.5 wt.%, and yet more preferably between 0.1 wt.% and 0.4 wt.% of a radical polymerisation inhibitor,
e) between 0.0005 wt.% and 0.02 wt.%, preferably between 0.0006 wt.% and 0.015 wt.%, more preferably between 0.00065 wt.% and 0.01 wt.%, of sulphur dioxide,
f) between 0 and 0.004 wt.%, preferably between 0.0001 wt.% and 0.003 wt.%, more preferably between 0.0005 wt.% and 0.0015 wt.% of boron trifluoride etherate, and
g) between 0 and 0.005 wt.%, preferably between 0.0005 wt.% and 0.003 wt.%, more preferably between 0.00075 wt.% and 0.0015 wt.% of methanesulfonic acid,
wherein the wt.% is based on the total weight of the composition.

In another preferred embodiment, the photocurable cyanoacrylate composition comprises:
a) between 65 wt.% and 90 wt.%, more preferably between 70 wt.% and 85 wt.%, more preferably between 75 wt.% and 80 wt.%, of a cyanoacrylate monomer, preferably selected from the group consisting of *n*-butyl 2-cyanoacrylate, *n*-hexyl 2-cyanoacrylate, 2-octyl 2-cyanoacrylate and mixtures thereof, more preferably the cyanoacrylate monomer is *n*-butyl 2-cyanoacrylate,
b) between 0.0055 wt.% and 0.0295 wt.% of ferrocene, preferably between 0.0075 wt.% and 0.0275 wt.%, more preferably between 0.015 wt.% and 0.025 wt.%, and yet more preferably it is 0.02 wt.%,
c) between 0.1 wt.% and 0.8 wt.%, preferably between 0.2 wt.% and 0.6 wt.%, more preferably between 0.24 wt.% and 0.44 wt.%, and yet more preferably between 0.3 wt.% and 0.4 wt.%, of a mixture of phenyl bis(2,4,6-trimethylbenzoyl)phosphine oxide (BAPO) and diphenyl (2,4,6-trimethylbenzoyl)phosphine oxide (TPO),
d) between 0.05 wt.% and 1.5 wt.%, preferably between 0.08 wt.% and 0.6 wt.%, more preferably between 0.09 wt.% and 0.5 wt.%, and yet more preferably between 0.1 wt.% and 0.4 wt.% of a radical polymerisation inhibitor,
e) between 0.0005 wt.% and 0.02 wt.%, preferably between 0.0006 wt.% and 0.015 wt.%, more preferably between 0.00065 wt.% and 0.01 wt.%, of sulphur dioxide,
f) between 0.0001 wt.% and 0.003 wt.%, more preferably between 0.0005 wt.% and 0.0015 wt.% of boron trifluoride etherate, and
g) between 0.0005 wt.% and 0.003 wt.%, more preferably between 0.00075 wt.% and 0.0015 wt.% of methanesulfonic acid,
wherein the wt.% is based on the total weight of the composition.

In another preferred embodiment, the photocurable cyanoacrylate composition comprises:
a) between 75 wt.% and 95 wt.%, more preferably between 80 wt.% and 90 wt.%, and more preferably between 83 wt.% and 87 wt.%, of a cyanoacrylate monomer, preferably selected from the group consisting of *n*-butyl 2-cyanoacrylate, *n*-hexyl 2-cyanoacrylate, *2*-octyl 2-cyanoacrylate and mixtures thereof, and more preferably the cyanoacrylate monomer is *n*-hexyl 2-cyanoacrylate,
b) between 0.0055 wt.% and 0.0295 wt.% of ferrocene, preferably between 0.0075 wt.% and 0.0275 wt.%, more preferably between 0.015 wt.% and 0.025 wt.%, and yet more preferably it is 0.02 wt.%,
c) between 0.1 wt.% and 0.8 wt.%, preferably between 0.2 wt.% and 0.6 wt.%, more preferably between 0.24 wt.% and 0.44 wt.%, and yet more preferably between 0.3 wt.% and 0.4 wt.% of diphenyl (2,4,6-trimethylbenzoyl)phosphine oxide (TPO),
d) between 0.05 wt.% and 1.5 wt.%, preferably between 0.08 wt.% and 0.6 wt.%, more preferably between 0.09 wt.% and 0.5 wt.%, and yet more preferably between 0.1 wt.% and 0.4 wt.% of a radical polymerisation inhibitor,
e) between 0.0005 wt.% and 0.02 wt.%, preferably between 0.0006 wt.% and 0.015 wt.%, more preferably between 0.00065 wt.% and 0.01 wt.%, of sulphur dioxide, and
f) between 0.0001 wt.% and 0.003 wt.%, more preferably between 0.0005 wt.% and 0.0015 wt.% of boron trifluoride etherate,
wherein the wt.% is based on the total weight of the composition.

The stability of the photocurable cyanoacrylate composition is confirmed by both real time and accelerated aging test detailed in the Examples section. Both the curing time and viscosity data indicate the stability of said composition.

### Syringe or cartridge

Also, part of the object of the invention is a syringe or a cartridge comprising photocurable cyanoacrylate composition.

### Use of the composition

The use of the photocurable composition for bonding substrates also forms part of the subject-matter of the invention.

The use of the photocurable composition as nail coating also forms part of the subject-matter of the invention.

A method for bonding substrates also forms part of the subject-matter of the invention. Said method for bonding substrates comprises the steps of:
1) applying the composition of the invention to at least one of the substrates,
2) placing together the substrates,
3) irradiating with a light of a wavelength comprised between 380 nm and 415 nm, and
4) maintaining the substrates clamped up to fixation.

The source of radiation emitting electromagnetic waves is selected from ultraviolet light, visible light, and a combination thereof.

As light source it may be employed LED irradiating in a wavelength comprised between 380 nm and 415 nm, having a potency comprised between 0.4 W and 36 W.

Another aspect of the invention is a photocurable cyanoacrylate composition for use as bioadhesive and biosealant. The composition is usually applied to the tissue as a liquid that then solidifies *in situ* by irradiating, providing intimate tissue contact.

A bioadhesive is a material that can be used as a 'glue' to hold tissue surfaces together. The adhesive can be placed between or over the tissues to be held in apposition until healing occurs.

A biosealant is a material that bonds to tissue surrounding an opening, such as a wound or incision, to halt the ingress or egress of liquid, gas or foreign matter.

In a preferred embodiment, the composition is for use in human or animal tissue bonding. In a preferred embodiment, the composition is applied topically. In a preferred embodiment, the composition is applied internally in procedures selected from the group consisting of cardiovascular, peripheral-vascular, orthopaedic, cardio-thoracic, gynaecological, neuro and general abdominal surgery.

The photocurable cyanoacrylate compositions prepared according to the present invention provide a controlled exotherm polymerisation with reduced temperature increase compared to the prior art. Targeted selection of photoinitiator, photoinitiator concentration range in combination with a proper stabilizer package provides stable photocurable formulations for different cyanoacrylate monomers, wherein polymerisation temperature remains below the threshold temperature to cause discomfort or damage to living tissues.

Such formulations have demonstrated, as well, performing with better T-peel adhesive strength in PVC substrates than the analogous formulations without controlled exotherm.

Additionally, the claimed composition allows the preparation of adhesives with optimal heat release, adequate viscosities and thixotropy which cover a wide range of viscosities presenting excellent physical and mechanical properties to be used as adhesive and sealants for industrial, consumer, medical and cosmetic applications in which controlled exotherm could be of interest.

Compositions of the invention provide unexpected advantages as tissue adhesive, which have demonstrated greater duration on the skin as wound sealant than other commercially available tissue adhesives showing no on demand photoinitiated polymerisation.

The inclusion in the composition of crosslinkers, such as difunctional acrylates or biscyanoacrylates for example, provide improved resistance in front of organic solvents, which could be relevant for applications such as tissue adhesives for both external and internal use, adhesives and sealants for medical device assembly, electronics and engineering.

### Examples

### Examples 1.1 to 1.8: Cyanoacrylate compositions

Cyanoacrylate compositions according to the invention were prepared by mixing monomer, acid stabilizers, radical scavengers, photoinitiator and ferrocene, according to the formulations shown in Tables I and II:

**TABLE I**

| Component/ Example | 1.1. | 1.2. | 1.3. | 1.4. |
|---|---|---|---|---|
| Monomer | ECA | BCA | HCA | OCA |
| | 98.78 | 98.98 | 99.1 | 99.37 |
| Sulphur dioxide | 0.0005 | 0.001 | 0.0005 | 0.01 |
| Boron trifluoride (etherate) | 0.0005 | 0.0005 | 0.0005 | -- |
| Butylated hydroxyanisole | 0.81 | 0.8 | 0.1 | -- |
| 2,2'-Methylenebis(4-methyl-6-tert-butylphenol) | 0.2 | -- | 0.5 | -- |
| Butylhydroxytoluene | -- | -- | -- | 0.2 |
| Hydroquinone | -- | -- | -- | -- |
| Diphenyl (2,4,6- trimethylbenzoyl)-phosphine oxide | 0.2 | 0.2 | 0.3 | 0.4 |
| Ferrocene | 0.02 | 0.02 | 0.02 | 0.02 |
| T Max. during polymerisation, ºC | 34 | 36 | 29 | 32 |

**TABLE 2**

| Component /Example | 1.5. | 1.6. | 1.7. | 1.8. |
|---|---|---|---|---|
| Monomer | MECA | ECA | ECA | ECA |
| | 99.27 | 98.58 | 99.38 | 99.39 |
| Sulphur dioxide | -- | 0.0005 | 0.0005 | 0.0005 |
| Boron trifluoride (etherate) | -- | 0.0005 | 0.0005 | 0.0005 |
| Butylated hydroxyanisole | 0.5 | -- | 0.2 | 0.2 |
| 2,2'-Methylenebis(4-methyl-6-tert-butylphenol) | -- | 1 | 0.2 | 0.2 |
| Butylhydroxytoluene | -- | -- | -- | -- |
| Hydroquinone | 0.02 | -- | -- | -- |
| Diphenyl (2,4,6- trimethylbenzoyl)-phosphine oxide | 0.2 | 0.4 | 0.2 | 0.2 |
| Ferrocene | 0.01 | 0.01 | 0.02 | 0.01 |
| T Max. during polymerisation, ºC | 34 | 43 | 43 | 33 |

wherein the following abbreviations were used: ethyl 2-cyanoacrylate (ECA), n-butyl 2-cyanoacrylate (BCA), n-hexyl 2-cyanoacrylate (HCA), 2-octyl 2-cyanoacrylate (OCA), 2-methoxyethyl 2-cyanoacrylate (MECA).

These compositions may include also other components such as, for, example, plasticizers, thickeners, thixotropic agents, accelerators, and mixtures thereof.

It can be observed that the maximum temperature reached during the polymerisation remained below 45 ºC, which is a temperature below the threshold temperature associated to discomfort or damage to living tissues.

### Examples 2.1 to 2.3: Cyanoacrylate compositions

Cyanoacrylate compositions according to the invention were prepared by mixing monomer, acid stabilizers, radical scavengers, photoinitiator, ferrocene, plasticizer, thickener and accelerator, according to the formulations shown in Table III:

**TABLE III**

| Component/ Example | 2.1. | 2.2. | 2.3. |
|---|---|---|---|
| Monomer | ECA | BCA | HCA |
| | 98.7 | 77.46 | 84.64 |
| Sulphur dioxide | 0.009 | 0.0065 | 0.01 |
| Boron trifluoride (etherate) | 0.002 | 0.0015 | 0.0005 |
| MSA | 0.001 | 0.001 | -- |
| Butylated hydroxyanisole | 0.21 | 0.1 | 0.1 |
| 2,2'-Methylenebis(4-methyl-6-tert-butylphenol) | 0.1 | 0.2 | -- |
| Butylhydroxytoluene | -- | 0.1 | -- |
| Glycerol triacetate | -- | 10 | -- |
| Propylene carbonate | -- | -- | 9.83 |
| Vinyl acetate/vinyl chloride copolymer | 7.5 | 8 | 5 |
| Fumed silica | -- | 4 | -- |
| Diphenyl (2,4,6- trimethylbenzoyl)-phosphine oxide | 0.2 | 0.2 | 0.4 |
| Phenyl bis (2,4,6- trimethylbenzoyl)-phosphine oxide | 0.075 | 0.1 | -- |
| Ferrocene | 0.02 | 0.02 | 0.02 |
| Crown ether | 0.2 | -- | -- |
| T Max. during polymerisation, ºC | 35 | 43 | 41 |

wherein the following abbreviations were used: ethyl cyanoacrylate (ECA), n-butyl cyanoacrylate (BCA), n-hexyl cyanoacrylate (HCA), methanesulfonic acid (MSA).

It can be observed that the maximum temperature reached during the polymerisation remained below 45 ºC, which is a temperature below the threshold temperature associated to discomfort or damage to living tissues.

The compositions were stable under accelerated aging tests at 60 ºC during 3 weeks. For example, the curing time of composition 2.3 remained practically unaltered after said test (43 seconds at t=0 vs. 44 seconds at t= 3 weeks). The viscosity remained substantially unchanged after the accelerated aging (1428 cP at t=0, and 1383 cP at t= 3 weeks).

### Examples 3.1 to 3.8: Cyanoacrylate compositions

Cyanoacrylate compositions according to the invention were prepared by mixing monomer, acid stabilizers, radical scavengers, photoinitiator, ferrocene, plasticizer, thickener, and crosslinkers, if present, according to the formulations shown in Tables IV and V:

**TABLE IV**

| Component/ Example | 3.1. | 3.2. | 3.3. | 3.4. |
|---|---|---|---|---|
| Monomer | BCA | BCA | BCA | BCA |
| | 84.64 | 76.2 | 67.7 | 67.7 |
| Sulphur dioxide | 0.01 | 0.009 | 0.008 | 0.008 |
| Boron trifluoride (etherate) | 0.0005 | 0.00045 | 0.0004 | 0.0004 |
| Butylated hydroxyanisole | 0.1 | 0.09 | 0.08 | 0.08 |
| Propylene carbonate | 9.83 | 9.25 | 8.22 | 8.22 |
| Vinyl acetate/vinyl chloride copolymer | 5 | 4,5 | 4 | 4 |
| Diphenyl (2,4,6- trimethylbenzoyl)-phosphine oxide | 0.4 | 0.36 | 0.32 | 0.32 |
| Ferrocene | 0.02 | 0.018 | 0.016 | 0.016 |
| TMPTA | -- | 7.2 | 19.2 | 14 |
| Bisphenol A ethoxylated diacrylate | -- | 2.4 | 3.2 | 4.6 |
| Bis cyanoacrylate | -- | -- | -- | 0.6 |
| T Max. during polymerisation, ºC | 36 | 39 | 39 | 39 |

**TABLE V**

| Component/ Example | 3.5. | 3.6. | 3.7. | 3.8. |
|---|---|---|---|---|
| Monomer | BCA | BCA | BCA | BCA |
| | 76.18 | 42.32 | 42.32 | 67.4 |
| Sulphur dioxide | 0.009 | 0.005 | 0.005 | 0.008 |
| Boron trifluoride (etherate) | 0.0005 | 0.0002 | 0.0002 | 0.0004 |
| Butylated hydroxyanisole | 0.09 | 0.05 | 0.05 | 0.08 |
| Propylene carbonate | 8.85 | 6.91 | 6.91 | 8.22 |
| Vinyl acetate/vinyl chloride copolymer | 4.5 | 2.5 | 2.5 | 4 |
| Diphenyl (2,4,6- trimethylbenzoyl)-phosphine oxide | 0.36 | 0.2 | 0.2 | 0.72 |
| Ferrocene | 0.018 | 0.01 | 0.01 | 0.016 |
| TMPTA | -- | 36 | 35 | 19.2 |
| Bisphenol A ethoxylated diacrylate | -- | 12 | 11.5 | 3.2 |
| Bis cyanoacrylate | 10 | -- | 1.5 | -- |
| T Max. during polymerisation, ºC | 36 | 39 | 39 | 39 |

wherein the following abbreviations were used: n-butyl cyanoacrylate (BCA), TMPTA (trimethylolpropane triacrylate).

It can be observed that the maximum temperature reached during the polymerisation remained below 45 ºC, which is a temperature below the threshold temperature associated to discomfort or damage to living tissues.

Compositions of Examples 3.2 to 3.8, all of them containing a crosslinking compound in the formulation, showed improved resistance to organic solvents (1 day in acetone at 25º C), in comparison to cyanoacrylate compositions, such as composition of Example 3.1, without crosslinking.

The compositions were stable under accelerated aging tests at 60 ºC during 3 weeks. For example, the curing time of composition 3.1 remained practically unaltered after said test (15 seconds at t=0 vs. 16 seconds at t= 3 weeks). The viscosity showed an initial increase during the accelerated aging, which stabilized later on (62 cP at t=0, 122 cP at t = 1 week, 161 cP at t= 2 weeks, and 161 at t= 3 weeks).

### Comparative Examples: Comparative cyanoacrylate compositions

Comparative cyanoacrylate compositions were prepared by mixing the components listed in Tables VI and VII:

**TABLE VI**

| Component/ Example | A | B | C | D |
|---|---|---|---|---|
| Monomer | ECA | ECA | ECA | ECA |
| | 99.78 | 99.88 | 93.3 | 99.37 |
| Sulphur dioxide | 0.0005 | 0.001 | 0.0005 | 0.0005 |
| Boron trifluoride (etherate) | 0.0005 | 0.0005 | 0.0005 | 0.0005 |
| Butylated hydroxyanisole | -- | -- | 0.8 | 0.2 |
| 2,2'-Methylenebis(4-methyl-6-tert-butylphenol) | -- | -- | 0.2 | 0.2 |
| Butylhydroxytoluene | -- | -- | -- | 0.2 |
| Diphenyl (2,4,6- trimethylbenzoyl)-phosphine oxide | 0.2 | 0.1 | 1.7 | 0.2 |
| Ferrocene | 0.01 | 0.01 | 0.02 | 0.03 |
| T Max. during polymerisation, ºC | 82 | 62 | 70 | 32 |

**TABLE VII**

| Component/ Example | E | F | G | H |
|---|---|---|---|---|
| Monomer | BCA | HCA | OCA | MECA |
| | 97.5 | 97.63 | 97.9 | 97.8 |
| Sulphur dioxide | 0.001 | 0.0005 | 0.01 | -- |
| Boron trifluoride (etherate) | 0.0005 | 0.0005 | -- | -- |
| Butylated hydroxyanisole | 0.8 | 0.1 | -- | 0.5 |
| 2,2'-Methylenebis(4-methyl-6-tert-butylphenol) | -- | 0.5 | -- | -- |
| Butylhydroxytoluene | -- | -- | 0.2 | -- |
| Hydroquinone | -- | -- | -- | 0.02 |
| Diphenyl (2,4,6- trimethylbenzoyl)-phosphine oxide | 1.7 | 1.8 | 1.9 | 1.7 |
| Ferrocene | 0.02 | 0.02 | 0.02 | 0.01 |
| T Max. during polymerisation, ºC | 62 | 51 | 62 | 81 |

It can be observed that the maximum temperature reached during the polymerisation of the comparative cyanoacrylate compositions exceeds 45 ºC, which is a temperature associated to discomfort or damage to living tissues.

### Example 4: Peel adhesion test of cyanoacrylate compositions

The peel adhesion test is used to determine the force required to de-bond two components joined by an adhesive. The test result, also known as bond strength, is generally represented as N (force to de-bond) / 25mm or 50mm (depending on specimen width). In 180-degree peel test, a constant 180º angle is maintained whilst the two glued components are peeled apart. The average load required to separate the two, over the length of the specimen is recorded and expressed as N/25mm.

100 µl of cyanoacrylate composition was placed on a PVC substrate of 2.5 cm x 10 cm. It was overlapped with another lap shear and clamped. It was irradiated 90 seconds with a LED lamp (405 nm), and the T-peel adhesion strength was measured using a peel tester model 1011 of company Instron Corporation.

Table VIII shows the results of the peel adhesion test 180º (mean value of three specimens, and standard deviation), expressed as N/25mm:

**TABLE VIII**

| Cyanoacrylate composition | Monomer | TPO/BAPO | T max. ºC | N/25mm |
|---|---|---|---|---|
| Example 1.1 | ECA | 2000 | 34 | 12.71 (5.2) |
| Comparative Example C | ECA | 17000 | 70 | 2.5 (1.5) |
| Example 3.1 | BCA | 4000 | 36 | 4.5 (3.3) |
| Comparative Example E | BCA | 17000 | 62 | 1.5 (90.7) |
| Example 1.5 | MECA | 2000 | 34 | 7.3 (3.74) |
| Comparative Example H | MECA | 17000 | 81 | 4.5 (4.6) |

It was observed that the cyanoacrylate compositions of the invention, with controlled exotherm, showed a better performance in peel adhesion test than compositions with higher maximum temperature reached during polymerisation.

### Example 5: Adhesion test of cyanoacrylate compositions

An adhesion test was performed with a cyanoacrylate composition of the invention, Example 3.1, with controlled exotherm (maximum temperature during polymerisation 36 ºC), and a commercial product based on methoxyethyl cyanoacrylate, without controlled exotherm (maximum temperature during polymerisation 73ºC).

25 µl of sample was placed on 1.25 cm² of a substrate contact surface. It was overlapped with another lap shear and clamped. A specimen was irradiated 10 seconds with a LED lamp (405 nm), and another specimen was kept 24 h dark at room temperature. The single joint lap-shear tensile strength was measured with a tester model 1011 of company Instron Corporation.

Table IX shows the results, expressed in Mpa, for different substrates (polymethylmethacrylate (PMMA), polycarbonate (PC) and polyvinyl chloride (PVC):

**TABLE IX**

| Substrate | Example 3.1 | | Comparative example | |
|---|---|---|---|---|
| | 24 h R.T. dark | After light irradiation | 24 h R.T. dark | After light irradiation |
| PMMA | 6.57* | 6.87 | 6.47* | 5.94 |
| PC | 9.65 | 9.70* | 5.91 | 7.18 |
| PVC | 5.05* | 9.65 | 7.93* | 4.95 |

| | | | | |
|---|---|---|---|---|
| (*) substrate failure | | | | |

It can be observed that the cyanoacrylate composition of the invention, with controlled exotherm, shows better adherence after light curing than the commercial product without controlled exotherm.

### Example 6: Adhesion test of cyanoacrylate compositions

An adhesion test was performed with a cyanoacrylate composition of the invention, Example 2.3, with controlled exotherm (maximum temperature during polymerisation 41 ºC), and a commercial product based on butyl cyanoacrylate, without no on-demand photoinitiated polymerisation.

Adhesion test was performed on skin of four animals [Species: Sus scrofa; Breed: Landrace x Largewhite; Source: Granja Mas el Cros; Sex: Female; Body weight range: 30 - 40 kg] identified with a number (1 to 4).

Surgery was practiced on day 0, and cyanoacrylate formulations were applied for wound closure.

Post-surgical control was carried out on days 1 to 31 after surgery. The animals were housed individually in 0.8 x 1.5 x 2 meters. The wounds were observed during the 1st, 2nd, 4th, 7th, 10th and 14th day (pigs 1 and 2) and in the 1st, 4th, 9th, 14th and 31st days (pigs 3 and 4) to observe the presence or absence of the adhesive or suture, the wound closure, inflammation and wound appearance. At the same time, biopsies were obtained in some cases for histological evaluation.

Both cyanoacrylate compositions showed similar results with complete wound healing and no wound dehiscence.

Cyanoacrylate composition of Example 2.3 showed a greater duration in the skin as wound sealant than commercial product.

Microscopically, wound healing was complete after 14 days of surgical incision and glue application.

## Claims

1. A photocurable cyanoacrylate composition, **characterized in that** it comprises:
a) at least 40 wt.% of a cyanoacrylate monomer,
b) between 0.0055 wt.% and 0.0295 wt.% of ferrocene,
c) between 0.1 wt.% and 0.8 wt.% of a benzoyl phosphine oxide as photoinitiator, preferably selected from phenyl bis(2,4,6-trimethylbenzoyl)phosphine oxide (BAPO), diphenyl (2,4,6-trimethylbenzoyl)phosphine oxide (TPO), benzoyl-diphenylphosphine oxide (BDPO), 2,4,6-trimethylbenzoyl-methoxy-phenylphosphine oxide (TMMPO), phthaloyl-bis(diphenylphosphine oxide) (PBDPO), 2,6-difluoro benzoyl-diphenylphosphine oxide (DFDPO), and mixtures thereof, more preferably selected from phenyl bis(2,4,6-trimethylbenzoyl)phosphine oxide (BAPO), diphenyl (2,4,6-trimethylbenzoyl)phosphine oxide (TPO), and mixtures thereof, and
d) between 0.05 wt.% and 1.5 wt.% of a radical polymerisation inhibitor,
wherein the wt.% is based on the total weight of the composition.

2. The photocurable cyanoacrylate composition according to claim 1, **characterized in that** the content of cyanoacrylate monomer is comprised between 65 wt.% and 95 wt.%, preferably between 70 wt.% and 90 wt.%, more preferably between 75 wt.% and 87 wt.%, and yet more preferably between 78 wt.% and 85 wt.%, wherein the wt.% is based on the total weight of the composition.

3. The photocurable cyanoacrylate composition according to anyone of claim 1 or 2, **characterized in that** the radical polymerisation inhibitor is selected from the group consisting of 4-methoxyphenol, hydroquinone, hydroquinone monomethyl ether, hydroxytoluene butyl ether, hydroxyanisole butyl ether, 2,2'-methylenebis(4-methyl-6-*tert-*butylphenol), 4,4'-methylenbis (2,6-di-*tert*-butylphenol) and mixtures thereof; preferably selected from the group consisting of hydroxyanisole butyl ether, 2,2'-methylenebis(4-methyl-6-*tert*-butylphenol), hydroxytoluene butyl ether, and mixtures thereof.

4. The photocurable cyanoacrylate composition according to anyone of claims 1 to 3, **characterized in that** the content of the radical polymerisation inhibitor is comprised between 0.08 wt.% and 0.6 wt.%, more preferably between 0.09 wt.% and 0.5 wt.%, and yet more preferably between 0.1 wt.% and 0.4 wt.%, wherein the wt.% is based on the total weight of the composition.

5. The photocurable cyanoacrylate composition according to anyone of claims 1 to 4, **characterized in that** the composition further comprises an acid stabilizing agent.

6. The photocurable cyanoacrylate composition according to claim 5, **characterized in that** the acid stabilizing agent is selected from the group consisting of boron trifluoride, boron trifluoride etherate, boron trifluoride dihydrate, trimethylsilyl triflate, sulphur dioxide, methanesulfonic acid, and mixtures thereof.

7. The photocurable cyanoacrylate composition according to anyone of claims 1 to 6, **characterized in that** it further contains one additive selected from thickening agents, plasticizers, thixotropic agents, crosslinking agents, accelerating agents, and mixtures thereof.

8. The photocurable cyanoacrylate composition according to claim 1, **characterized in that** it comprises:
a) at least 40 wt.%, preferably between 65 wt.% and 95 wt.% of a cyanoacrylate monomer,
b) between 0.0055 wt.% and 0.0295 wt.% of ferrocene, preferably between 0.0075 wt.% and 0.0275 wt.%, more preferably between 0.015 wt.% and 0.025 wt.%, and yet more preferably it is 0.02 wt.%
c) between 0.1 wt.% and 0.8 wt.%, preferably between 0.2 wt.% and 0.6 wt.%, more preferably between 0.24 wt.% and 0.44 wt.%, and yet more preferably between 0.3 wt.% and 0.4 wt.% of a benzoyl phosphine oxide as photoinitiator, preferably selected from phenyl bis(2,4,6-trimethylbenzoyl)phosphine oxide (BAPO), diphenyl (2,4,6-trimethylbenzoyl)phosphine oxide (TPO), benzoyl-diphenylphosphine oxide (BDPO), 2,4,6-trimethylbenzoyl-methoxy-phenylphosphine oxide (TMMPO), phthaloyl-bis(diphenylphosphine oxide) (PBDPO), 2,6-difluoro benzoyl-diphenylphosphine oxide (DFDPO), and mixtures thereof, more preferably selected from phenyl bis(2,4,6-trimethylbenzoyl)phosphine oxide (BAPO), diphenyl (2,4,6-trimethylbenzoyl)phosphine oxide (TPO), and mixtures thereof,
d) between 0.05 wt.% and 1.5 wt.%, preferably between 0.08 wt.% and 0.6 wt.%, more preferably between 0.09 wt.% and 0.5 wt.%, and yet more preferably between 0.1 wt.% and 0.4 wt.% of a radical polymerisation inhibitor,
e) between 0.0005 wt.% and 0.02 wt.%, preferably between 0.0006 wt.% and 0.015 wt.%, more preferably between 0.00065 wt.% and 0.01 wt.%, of sulphur dioxide,
f) between 0 and 0.004 wt.%, preferably between 0.0001 wt.% and 0.003 wt.%, more preferably between 0.0005 wt.% and 0.0015 wt.% of boron trifluoride etherate, and
g) between 0 and 0.005 wt.%, preferably between 0.0005 wt.% and 0.003 wt.%, more preferably between 0.00075 wt.% and 0.0015 wt.% of methanesulfonic acid,
wherein the wt.% is based on the total weight of the composition.

9. The photocurable cyanoacrylate composition according to claim 1, **characterized in that** it comprises:
a) between 65 wt.% and 90 wt.%, more preferably between 70 wt.% and 85 wt.%, more preferably between 75 wt.% and 80 wt.%, of a cyanoacrylate monomer, preferably selected from the group consisting of *n*-butyl 2-cyanoacrylate, *n*-hexyl 2-cyanoacrylate, *2*-octyl 2-cyanoacrylate and mixtures thereof, more preferably the cyanoacrylate monomer is *n*-butyl 2-cyanoacrylate,
b) between 0.0055 wt.% and 0.0295 wt.% of ferrocene, preferably between 0.0075 wt.% and 0.0275 wt.%, more preferably between 0.015 wt.% and 0.025 wt.%, and yet more preferably it is 0.02 wt.%,
c) between 0.1 wt.% and 0.8 wt.%, preferably 0.2 wt.% and 0.6 wt.%, more preferably between 0.24 wt.% and 0.44 wt.%, and yet more preferably between 0.3 wt.% and 0.4 wt.%, of a mixture of phenyl bis(2,4,6-trimethylbenzoyl)phosphine oxide (BAPO) and diphenyl (2,4,6-trimethylbenzoyl)phosphine oxide (TPO),
d) between 0.05 wt.% and 1.5 wt.%, preferably between 0.08 wt.% and 0.6 wt.%, more preferably between 0.09 wt.% and 0.5 wt.%, and yet more preferably between 0.1 wt.% and 0.4 wt.% of a radical polymerisation inhibitor,
e) between 0.0005 wt.% and 0.02 wt.%, preferably between 0.0006 wt.% and 0.015 wt.%, more preferably between 0.00065 wt.% and 0.01 wt.%, of sulphur dioxide,
f) between 0.0001 wt.% and 0.003 wt.%, more preferably between 0.0005 wt.% and 0.0015 wt.% of boron trifluoride etherate, and
g) between 0.0005 wt.% and 0.003 wt.%, more preferably between 0.00075 wt.% and 0.0015 wt.% of methanesulfonic acid,
wherein the wt.% is based on the total weight of the composition.

10. The photocurable cyanoacrylate composition according to claim 1, **characterized in that** it comprises:
a) between 75 wt.% and 95 wt.%, more preferably between 80 wt.% and 90 wt.%, and more preferably between 83 wt.% and 87 wt.%, of a cyanoacrylate monomer, preferably selected from the group consisting of *n*-butyl 2-cyanoacrylate, *n*-hexyl 2-cyanoacrylate, *2*-octyl 2-cyanoacrylate and mixtures thereof, and more preferably the cyanoacrylate monomer is *n*-hexyl 2-cyanoacrylate,
b) between 0.0055 wt.% and 0.0295 wt.% of ferrocene, preferably between 0.0075 wt.% and 0.0275 wt.%, more preferably between 0.015 wt.% and 0.025 wt.%, and yet more preferably it is 0.02 wt.%,
c) between 0.1 wt.% and 0.8 wt.%, preferably 0.2 wt.% and 0.6 wt.%, more preferably between 0.24 wt.% and 0.44 wt.%, and yet more preferably between 0.3 wt.% and 0.4 wt.% of diphenyl (2,4,6-trimethylbenzoyl)phosphine oxide (TPO),
d) between 0.05 wt.% and 1.5 wt.%, preferably between 0.08 wt.% and 0.6 wt.%, more preferably between 0.09 wt.% and 0.5 wt.%, and yet more preferably between 0.1 wt.% and 0.4 wt.% of a radical polymerisation inhibitor,
e) between 0.0005 wt.% and 0.02 wt.%, preferably between 0.0006 wt.% and 0.015 wt.%, more preferably between 0.00065 wt.% and 0.01 wt.%, of sulphur dioxide, and
f) between 0.0001 wt.% and 0.003 wt.%, more preferably between 0.0005 wt.% and 0.0015 wt.% of boron trifluoride etherate,
wherein the wt.% is based on the total weight of the composition.

11. A syringe or a cartridge comprising the photocurable cyanoacrylate composition of any one of claims 1 to 10.

12. The photocurable cyanoacrylate composition of any one of claims 1 to 10 for use in bonding substrates.

13. The use of the photocurable cyanoacrylate composition of any one of claims 1 to 10 as nail coating.

14. The photocurable cyanoacrylate composition of any of claims 1 to 10 for use as bioadhesive and biosealant.

15. The photocurable cyanoacrylate composition of any one of claims 1 to 10 for use in a method for bonding substrates, **characterized in that** it comprises the steps of:
1) applying the composition of any one of claims 1 to 10 to at least one of the substrates,
2) placing together the substrates,
3) irradiating with a light of a wavelength comprised between 380 nm and 415 nm, and
4) maintaining the substrates clamped up to fixation.

## Patentansprüche

1. Photohärtbare Cyanoacrylat-Zusammensetzung, **dadurch gekennzeichnet, dass** sie umfasst:
a) mindestens 40 Gew.-% eines Cyanoacrylatmonomers,
b) zwischen 0,0055 Gew.-% und 0,0295 Gew.-% Ferrocen,
c) zwischen 0,1 Gew.-% und 0,8 Gew.-% eines Benzoylphosphinoxids als Photoinitiator, vorzugsweise ausgewählt aus Phenyl-bis(2,4,6-trimethylbenzoyl)-phosphinoxid (BAPO), Diphenyl(2,4,6-trimethylbenzoyl)phosphinoxid (TPO), Benzoyldiphenylphosphinoxid (BDPO), 2,4,6-Trimethylbenzoyl-methoxy-phenylphosphinoxid (TMMPO), Phthaloyl-bis(diphenylphosphinoxid) (PBDPO), 2,6-Difluorbenzoyldiphenylphosphinoxid (DFDPO) und Mischungen davon, bevorzugt ausgewählt aus Phenyl-bis(2,4,6-trimethylbenzoyl)phosphinoxid (BAPO), Diphenyl(2,4,6-trimethylbenzoyl)phosphinoxid (TPO) und Mischungen davon, und
d) zwischen 0,05 Gew.-% und 1,5 Gew.-% eines radikalischen Polymerisationsinhibitors, wobei die Gew.-% auf das Gesamtgewicht der Zusammensetzung bezogen sind.

2. Photohärtbare Cyanoacrylatzusammensetzung nach Anspruch 1, **dadurch gekennzeichnet, dass** der Gehalt an Cyanoacrylatmonomer zwischen 65 Gew.-% und 95 Gew.-%, vorzugsweise zwischen 70 Gew.-% und 90 Gew.-%, bevorzugter zwischen 75 Gew.-% und 87 Gew.-% und noch bevorzugter zwischen 78 Gew.-% und 85 Gew.-% liegt, wobei die Gew.-% auf das Gesamtgewicht der Zusammensetzung bezogen sind.

3. Photohärtbare Cyanoacrylat-Zusammensetzung nach einem der Ansprüche 1 oder 2, **dadurch gekennzeichnet, dass** der radikalische Polymerisationsinhibitor ausgewählt ist aus der Gruppe bestehend aus 4-Methoxyphenol, Hydrochinon, Hydrochinonmonomethylether, Hydroxytoluolbutylether, Hydroxyanisolbutylether, 2,2'-Methylenbis(4-methyl-6-tert-butylphenol), 4,4'-Methylenbis(2,6-di-*tert*-butylphenol) und Mischungen davon; vorzugsweise ausgewählt aus der Gruppe bestehend aus Hydroxyanisolbutylether, 2,2'-Methylenbis(4-methyl-6-*tert*-butylphenol), Hydroxytoluolbutylether und Mischungen davon.

4. Photohärtbare Cyanoacrylat-Zusammensetzung nach einem der Ansprüche 1 bis 3, **dadurch gekennzeichnet, dass** der Gehalt an radikalischem Polymerisationsinhibitor zwischen 0,08 Gew.-% und 0,6 Gew.-%, bevorzugter zwischen 0,09 Gew.-% und 0,5 Gew.-% und noch bevorzugter zwischen 0,1 Gew.-% und 0,4 Gew.-% liegt, wobei die Gew.-% auf das Gesamtgewicht der Zusammensetzung bezogen sind.

5. Photohärtbare Cyanoacrylat-Zusammensetzung nach einem der Ansprüche 1 bis 4, **dadurch gekennzeichnet, dass** die Zusammensetzung außerdem ein Säurestabilisierungsmittel umfasst.

6. Photohärtbare Cyanoacrylat-Zusammensetzung nach Anspruch 5, **dadurch gekennzeichnet, dass** das Säurestabilisierungsmittel ausgewählt ist aus der Gruppe bestehend aus Bortrifluorid, Bortrifluoridetherat, Bortrifluoriddihydrat, Trimethylsilyltriflat, Schwefeldioxid, Methansulfonsäure und Mischungen davon.

7. Photohärtbare Cyanoacrylat-Zusammensetzung nach einem der Ansprüche 1 bis 6, **dadurch gekennzeichnet, dass** sie außerdem ein Additiv enthält, das aus Verdickungsmitteln, Weichmachern, Thixotropiermitteln, Vernetzungsmitteln, Beschleunigern und Mischungen davon ausgewählt ist.

8. Photohärtbare Cyanoacrylat-Zusammensetzung nach Anspruch 1, **dadurch gekennzeichnet, dass** sie umfasst:
a) mindestens 40 Gew.-%, vorzugsweise zwischen 65 Gew.-% und 95 Gew.-% eines Cyanoacrylatmonomers,
b) zwischen 0,0055 Gew.-% und 0,0295 Gew.-% Ferrocen, vorzugsweise zwischen 0,0075 Gew.-% und 0,0275 Gew.-%, bevorzugter zwischen 0,015 Gew.-% und 0,025 Gew.-%, und noch bevorzugter beträgt sie 0,02 Gew.-%
c) zwischen 0,1 Gew.-% und 0,8 Gew.-%, vorzugsweise zwischen 0,2 Gew.-% und 0,6 Gew.-%, bevorzugter zwischen 0,24 Gew.-% und 0,44 Gew.-%, und noch bevorzugter zwischen 0,3 Gew.-% und 0,4 Gew.-% eines Benzoylphosphinoxids als Photoinitiator, vorzugsweise ausgewählt aus Phenyl-bis(2,4,6-trimethylbenzoyl)phosphinoxid (BAPO), Diphenyl(2,4,6-trimethylbenzoyl)phosphinoxid (TPO), Benzoyl-diphenylphosphinoxid (BDPO), 2,4,6-Trimethylbenzoyl-methoxy-phenylphosphinoxid (TMMPO), Phthaloyl-bis(diphenylphosphinoxid) (PBDPO), 2,6-Difluorbenzoyldiphenylphosphinoxid (DFDPO) und Mischungen davon, vorzugsweise ausgewählt aus Phenyl-bis(2,4,6-trimethylbenzoyl)phosphinoxid (BAPO), Diphenyl(2,4,6-trimethylbenzoyl)phosphinoxid (TPO) und Mischungen davon,
d) zwischen 0,05 Gew.-% und 1,5 Gew.-%, vorzugsweise zwischen 0,08 Gew.-% und 0,6 Gew.-%, bevorzugter zwischen 0,09 Gew.-% und 0,5 Gew.-% und noch bevorzugter zwischen 0,1 Gew.-% und 0,4 Gew.-% eines radikalischen Polymerisationsinhibitors,
e) zwischen 0,0005 Gew.-% und 0,02 Gew.-%, vorzugsweise zwischen 0,0006 Gew.-% und 0,015 Gew.-%, bevorzugter zwischen 0,00065 Gew.-% und 0,01 Gew.-%, Schwefeldioxid,
f) zwischen 0 und 0,004 Gew.-%, vorzugsweise zwischen 0,0001 Gew.-% und 0,003 Gew.-%, bevorzugter zwischen 0,0005 Gew.-% und 0,0015 Gew.-% Bortrifluoridetherat, und
g) zwischen 0 und 0,005 Gew.-%, vorzugsweise zwischen 0,0005 Gew.-% und 0,003 Gew.-%, bevorzugter zwischen 0,00075 Gew.-% und 0,0015 Gew.-% Methansulfonsäure,
wobei sich die Gew.-% auf das Gesamtgewicht der Zusammensetzung beziehen.

9. Photohärtbare Cyanoacrylat-Zusammensetzung nach Anspruch 1, **dadurch gekennzeichnet, dass** sie umfasst:
a) zwischen 65 Gew.-% und 90 Gew.-%, bevorzugter zwischen 70 Gew.-% und 85 Gew.-%, bevorzugter zwischen 75 Gew.-% und 80 Gew.-%.% eines Cyanoacrylatmonomers, vorzugsweise ausgewählt aus der Gruppe bestehend aus *n*-Butyl-2-cyanoacrylat, *n*-Hexyl-2-cyanoacrylat, 2-Octyl-2-cyanoacrylat und Mischungen davon, wobei das Cyanoacrylatmonomer bevorzugter *n*-Butyl-2-cyanoacrylat ist,
b) zwischen 0,0055 Gew.-% und 0,0295 Gew.-% Ferrocen, vorzugsweise zwischen 0,0075 Gew.-% und 0,0275 Gew.-%, bevorzugter zwischen 0,015 Gew.-% und 0,025 Gew.-%, und noch bevorzugter beträgt sie 0,02 Gew.-%,
c) zwischen 0,1 Gew.-% und 0,8 Gew.-%, vorzugsweise zwischen 0,2 Gew.-% und 0,6 Gew.-%, bevorzugter zwischen 0,24 Gew.-% und 0,44 Gew.-%, und noch bevorzugter zwischen 0.3 Gew.-% und 0,4 Gew.-% einer Mischung aus Phenylbis(2,4,6-trimethylbenzoyl)phosphinoxid (BAPO) und Diphenyl(2,4,6-trimethylbenzoyl)phosphinoxid (TPO),
d) zwischen 0,05 Gew.-% und 1,5 Gew.-%, vorzugsweise zwischen 0,08 Gew.-% und 0,6 Gew.-%, bevorzugter zwischen 0,09 Gew.-% und 0,5 Gew.-% und noch bevorzugter zwischen 0,1 Gew.-% und 0,4 Gew.-% eines radikalischen Polymerisationsinhibitors,
e) zwischen 0,0005 Gew.-% und 0,02 Gew.-%, vorzugsweise zwischen 0,0006 Gew.-% und 0,015 Gew.-%, bevorzugter zwischen 0,00065 Gew.-% und 0,01 Gew.-%, Schwefeldioxid,
f) zwischen 0,0001 Gew.-% und 0,003 Gew.-%, bevorzugter zwischen 0,0005 Gew.-% und 0,0015 Gew.-% Bortrifluoridetherat, und
g) zwischen 0,0005 Gew.-% und 0,003 Gew.-%, bevorzugter zwischen 0,00075 Gew.-% und 0,0015 Gew.-% Methansulfonsäure,
wobei sich die Gew.-% auf das Gesamtgewicht der Zusammensetzung beziehen.

10. Photohärtbare Cyanoacrylat-Zusammensetzung nach Anspruch 1, **dadurch gekennzeichnet, dass** sie umfasst:
a) zwischen 75 Gew.-% und 95 Gew.-%, bevorzugter zwischen 80 Gew.-% und 90 Gew.-%, und bevorzugter zwischen 83 Gew.-% und 87 Gew.-%.% eines Cyanoacrylatmonomers, vorzugsweise ausgewählt aus der Gruppe bestehend aus *n*-Butyl-2-cyanoacrylat, *n*-Hexyl-2-cyanoacrylat, 2-Octyl-2-cyanoacrylat und Mischungen davon, und wobei das Cyanoacrylatmonomer bevorzugter *n*-Butyl-2-cyanoacrylat ist,
b) zwischen 0,0055 Gew.-% und 0,0295 Gew.-% Ferrocen, vorzugsweise zwischen 0,0075 Gew.-% und 0,0275 Gew.-%, bevorzugter zwischen 0,015 Gew.-% und 0,025 Gew.-%, und noch bevorzugter beträgt sie 0,02 Gew.-%,
c) zwischen 0,1 Gew.-% und 0,8 Gew.-%, vorzugsweise zwischen 0,2 Gew.-% und 0,6 Gew.-%, bevorzugter zwischen 0,24 Gew.-% und 0,44 Gew.-% und noch bevorzugter zwischen 0,3 Gew.-% und 0,4 Gew.-% Diphenyl(2,4,6-trimethylbenzoyl)phosphinoxid (TPO),
d) zwischen 0,05 Gew.-% und 1,5 Gew.-%, vorzugsweise zwischen 0,08 Gew.-% und 0,6 Gew.-%, bevorzugter zwischen 0,09 Gew.-% und 0,5 Gew.-% und noch bevorzugter zwischen 0,1 Gew.-% und 0,4 Gew.-% eines radikalischen Polymerisationsinhibitors,
e) zwischen 0,0005 Gew.-% und 0,02 Gew.-%, vorzugsweise zwischen 0,0006 Gew.-% und 0,015 Gew.-%, bevorzugter zwischen 0,00065 Gew.-% und 0,01 Gew.-% Schwefeldioxid, und
f) zwischen 0,0001 Gew.-% und 0,003 Gew.-%, bevorzugter zwischen 0,0005 Gew.-% und 0,0015 Gew.-% Bortrifluoridetherat,
wobei sich die Gew.-% auf das Gesamtgewicht der Zusammensetzung beziehen.

11. Spritze oder Kartusche, die die photohärtbare Cyanoacrylat-Zusammensetzung nach einem der Ansprüche 1 bis 10 umfasst.

12. Photohärtbare Cyanoacrylat-Zusammensetzung nach einem der Ansprüche 1 bis 10 zur Verwendung beim Verkleben von Substraten.

13. Verwendung der photohärtbaren Cyanoacrylat-Zusammensetzung nach einem der Ansprüche 1 bis 10 als Nagelbeschichtung.

14. Photohärtbare Cyanoacrylat-Zusammensetzung nach einem der Ansprüche 1 bis 10 zur Verwendung als Bioklebstoff und Bioversiegelung.

15. Photohärtbare Cyanoacrylat-Zusammensetzung nach einem der Ansprüche 1 bis 10 zur Verwendung in einem Verfahren zum Verbinden von Substraten, **dadurch gekennzeichnet, dass** es die folgenden Schritte umfasst:
1) Aufbringen der Zusammensetzung nach einem der Ansprüche 1 bis 10 auf mindestens eines der Substrate,
2) Zusammenfügen der Substrate,
3) Bestrahlen mit einem Licht einer Wellenlänge, die zwischen 380 nm und 415 nm liegt, und
4) Festklemmen der Substrate bis zur Fixierung.

## Revendications

1. Composition de cyanoacrylate photodurcissable, **caractérisée en ce qu'**elle comprend :
a) au moins 40 % en poids d'un monomère cyanoacrylate,
b) entre 0,0055 % en poids et 0,0295 % en poids de ferrocène,
c) entre 0,1 % en poids et 0,8 % en poids d'un oxyde de benzoylphosphine en tant que photoinitiateur, de préférence choisi parmi l'oxyde de phénylbis(2,4,6-triméthylbenzoyl)phosphine (BAPO), l'oxyde de diphényl(2,4,6-triméthylbenzoyl)phosphine (TPO), l'oxyde de benzoyldiphénylphosphine (BDPO), l'oxyde de 2,4,6-triméthylbenzoylméthoxyphénylphosphine (TMMPO), le phtaloylbis(oxyde de diphénylphosphine) (PBDPO), l'oxyde de 2,6-difluorobenzoyldiphénylphosphine (DFDPO) et les mélanges de ceux-ci, plus préférablement choisi parmi l'oxyde de phénylbis(2,4,6-triméthylbenzoyl)phosphine (BAPO), l'oxyde de diphényl(2,4,6-triméthylbenzoyl)phosphine (TPO) et les mélanges de ceux-ci, et
d) entre 0,05 % en poids et 1,5 % en poids d'un inhibiteur de polymérisation radicalaire, le % en poids étant basé sur le poids total de la composition.

2. Composition de cyanoacrylate photodurcissable selon la revendication 1, **caractérisée en ce que** la teneur en monomère cyanoacrylate est comprise entre 65 % en poids et 95 % en poids, de préférence entre 70 % en poids et 90 % en poids, plus préférablement entre 75 % en poids et 87 % en poids et encore plus préférablement entre 78 % en poids et 85 % en poids, le % en poids étant basé sur le poids total de la composition.

3. Composition de cyanoacrylate photodurcissable selon l'une quelconque des revendications 1 ou 2, **caractérisée en ce que** l'inhibiteur de polymérisation radicalaire est choisi dans le groupe constitué par le 4-méthoxyphénol, l'hydroquinone, l'éther monométhylique de l'hydroquinone, l'éther butylique de l'hydroxytoluène, l'éther butylique de l'hydroxyanisole, le 2,2'-méthylènebis(4-méthyl-6-*tert*-butylphénol), le 4,4'-méthylènebis(2,6-di-*tert*-butylphénol) et les mélanges de ceux-ci ; de préférence choisi dans le groupe constitué par l'éther butylique de l'hydroxyanisole, le 2,2'-méthylènebis(4-méthyl-6-*tert*-butylphénol), l'éther butylique de l'hydroxytoluène et les mélanges de ceux-ci.

4. Composition de cyanoacrylate photodurcissable selon l'une quelconque des revendications 1 à 3, **caractérisée en ce que** la teneur de l'inhibiteur de polymérisation radicalaire est comprise entre 0,08 % en poids et 0,6 % en poids, plus préférablement entre 0,09 % en poids et 0,5 % en poids et encore plus préférablement entre 0,1 % en poids et 0,4 % en poids, le % en poids étant basé sur le poids total de la composition.

5. Composition de cyanoacrylate photodurcissable selon l'une quelconque des revendications 1 à 4, **caractérisée en ce que** la composition comprend en outre un agent stabilisant acide.

6. Composition de cyanoacrylate photodurcissable selon la revendication 5, **caractérisée en ce que** l'agent stabilisant acide est choisi dans le groupe constitué par le trifluorure de bore, l'éthérate de trifluorure de bore, le trifluorure de bore dihydraté, le triflate de triméthylsilyle, le dioxyde de soufre, l'acide méthanesulfonique et les mélanges de ceux-ci.

7. Composition de cyanoacrylate photodurcissable selon l'une quelconque des revendications 1 à 6, **caractérisée en ce qu'**elle contient en outre un additif choisi parmi les agents épaississants, les plastifiants, les agents thixotropes, les agents de réticulation, les agents accélérateurs et les mélanges de ceux-ci.

8. Composition de cyanoacrylate photodurcissable selon la revendication 1, **caractérisée en ce qu'**elle comprend :
a) au moins 40 % en poids, de préférence entre 65 % en poids et 95 % en poids d'un monomère cyanoacrylate,
b) entre 0,0055 % en poids et 0,0295 % en poids de ferrocène, de préférence entre 0,0075 % en poids et 0,0275 % en poids, plus préférablement entre 0,015 % en poids et 0,025 % en poids et encore plus préférablement 0,02 % en poids
c) entre 0,1 % en poids et 0,8 % en poids, de préférence entre 0,2 % en poids et 0,6 % en poids, plus préférablement entre 0,24 % en poids et 0,44 % en poids et encore plus préférablement entre 0,3 % en poids et 0,4 % en poids d'un oxyde de benzoylphosphine en tant que photoinitiateur, de préférence choisi parmi l'oxyde de phénylbis(2,4,6-triméthylbenzoyl)phosphine (BAPO), l'oxyde de diphényl(2,4,6-triméthylbenzoyl)phosphine (TPO), l'oxyde de benzoyldiphénylphosphine (BDPO), l'oxyde de 2,4,6-triméthylbenzoylméthoxyphénylphosphine (TMMPO), le phtaloylbis(oxyde de diphénylphosphine) (PBDPO), l'oxyde de 2,6-difluorobenzoyldiphénylphosphine (DFDPO) et les mélanges de ceux-ci, plus préférablement choisi parmi l'oxyde de phénylbis(2,4,6-triméthylbenzoyl)phosphine (BAPO), l'oxyde de diphényl(2,4,6-triméthylbenzoyl)phosphine (TPO) et les mélanges de ceux-ci,
d) entre 0,05 % en poids et 1,5 % en poids, de préférence entre 0,08 % en poids et 0,6 % en poids, plus préférablement entre 0,09 % en poids et 0,5 % en poids et encore plus préférablement entre 0,1 % en poids et 0,4 % en poids d'un inhibiteur de polymérisation radicalaire,
e) entre 0,0005 % en poids et 0,02 % en poids, de préférence entre 0,0006 % en poids et 0,015 % en poids, plus préférablement entre 0,00065 % en poids et 0,01 % en poids, de dioxyde de soufre,
f) entre 0 et 0,004 % en poids, de préférence entre 0,0001 % en poids et 0,003 % en poids, plus préférablement entre 0,0005 % en poids et 0,0015 % en poids d'éthérate de trifluorure de bore et
g) entre 0 et 0,005 % en poids, de préférence entre 0,0005 % en poids et 0,003 % en poids, plus préférablement entre 0,00075 % en poids et 0,0015 % en poids d'acide méthanesulfonique,
le % en poids étant basé sur le poids total de la composition.

9. Composition de cyanoacrylate photodurcissable selon la revendication 1, **caractérisée en ce qu'**elle comprend :
a) entre 65 % en poids et 90 % en poids, plus préférablement entre 70 % en poids et 85 % en poids, plus préférablement entre 75 % en poids et 80 % en poids, d'un monomère cyanoacrylate, de préférence choisi dans le groupe constitué par le 2-cyanoacrylate de *n*-butyle, le 2-cyanoacrylate de n-hexyle, le 2-cyanoacrylate de 2-octyle et les mélanges de ceux-ci, plus préférablement le monomère cyanoacrylate étant le 2-cyanoacrylate de *n-*butyle,
b) entre 0,0055 % en poids et 0,0295 % en poids de ferrocène, de préférence entre 0,0075 % en poids et 0,0275 % en poids, plus préférablement entre 0,015 % en poids et 0,025 % en poids et encore plus préférablement 0,02 % en poids,
c) entre 0,1 % en poids et 0,8 % en poids, de préférence 0,2 % en poids et 0,6 % en poids, plus préférablement entre 0,24 % en poids et 0,44 % en poids et encore plus préférablement entre 0,3 % en poids et 0,4 % en poids, d'un mélange d'oxyde de phénylbis(2,4,6-triméthylbenzoyl)phosphine (BAPO) et d'oxyde de diphényl(2,4,6-triméthylbenzoyl)phosphine (TPO),
d) entre 0,05 % en poids et 1,5 % en poids, de préférence entre 0,08 % en poids et 0,6 % en poids, plus préférablement entre 0,09 % en poids et 0,5 % en poids et encore plus préférablement entre 0,1 % en poids et 0,4 % en poids d'un inhibiteur de polymérisation radicalaire,
e) entre 0,0005 % en poids et 0,02 % en poids, de préférence entre 0,0006 % en poids et 0,015 % en poids, plus préférablement entre 0,00065 % en poids et 0,01 % en poids, de dioxyde de soufre,
f) entre 0,0001 % en poids et 0,003 % en poids, plus préférablement entre 0,0005 % en poids et 0,0015 % en poids d'éthérate de trifluorure de bore et
g) entre 0,0005 % en poids et 0,003 % en poids, plus préférablement entre 0,00075 % en poids et 0,0015 % en poids d'acide méthanesulfonique,
le % en poids étant basé sur le poids total de la composition.

10. Composition de cyanoacrylate photodurcissable selon la revendication 1, **caractérisée en ce qu'**elle comprend :
a) entre 75 % en poids et 95 % en poids, plus préférablement entre 80 % en poids et 90 % en poids et plus préférablement entre 83 % en poids et 87 % en poids, d'un monomère cyanoacrylate, de préférence choisi dans le groupe constitué par le 2-cyanoacrylate de *n*-butyle, le 2-cyanoacrylate de *n*-hexyle, le 2-cyanoacrylate de 2-octyle et les mélanges de ceux-ci et plus préférablement le monomère cyanoacrylate étant le 2-cyanoacrylate de *n-*hexyle,
b) entre 0,0055 % en poids et 0,0295 % en poids de ferrocène, de préférence entre 0,0075 % en poids et 0,0275 % en poids, plus préférablement entre 0,015 % en poids et 0,025 % en poids et encore plus préférablement 0,02 % en poids,
c) entre 0,1 % en poids et 0,8 % en poids, de préférence 0,2 % en poids et 0,6 % en poids, plus préférablement entre 0,24 % en poids et 0,44 % en poids, et encore plus préférablement entre 0,3 % en poids et 0,4 % en poids d'oxyde de diphényl(2,4,6-triméthylbenzoyl) phosphine (TPO),
d) entre 0,05 % en poids et 1,5 % en poids, de préférence entre 0,08 % en poids et 0,6 % en poids, plus préférablement entre 0,09 % en poids et 0,5 % en poids et encore plus préférablement entre 0,1 % en poids et 0,4 % en poids d'un inhibiteur de polymérisation radicalaire,
e) entre 0,0005 % en poids et 0,02 % en poids, de préférence entre 0,0006 % en poids et 0,015 % en poids, plus préférablement entre 0,00065 % en poids et 0,01 % en poids, de dioxyde de soufre et
f) entre 0,0001 % en poids et 0,003 % en poids, plus préférablement entre 0,0005 % en poids et 0,0015 % en poids d'éthérate de trifluorure de bore,
le % en poids étant basé sur le poids total de la composition.

11. Seringue ou cartouche comprenant la composition de cyanoacrylate photodurcissable selon l'une quelconque des revendications 1 à 10.

12. Composition de cyanoacrylate photodurcissable selon l'une quelconque des revendications 1 à 10 destinée à être utilisée en collage de substrats.

13. Utilisation de la composition de cyanoacrylate photodurcissable selon l'une quelconque des revendications 1 à 10 en tant que vernis à ongles.

14. Composition de cyanoacrylate photodurcissable selon l'une quelconque des revendications 1 à 10 destinée à être utilisée en tant que bioadhésif et bioscellant.

15. Composition de cyanoacrylate photodurcissable selon l'une quelconque des revendications 1 à 10 destinée à être utilisée dans un procédé de collage de substrats, **caractérisé en ce qu'**il comprend les étapes consistant à :
1) appliquer la composition selon l'une quelconque des revendications 1 à 10 sur au moins l'un des substrats,
2) assembler les substrats,
3) exposer à une lumière ayant une longueur d'onde comprise entre 380 nm et 415 nm et
4) maintenir les substrats serrés jusqu'à la fixation.
